# EUROPEAN PATENT APPLICATION

(11) **EP 1 302 200 A1**
(43) Date of publication of application: **16.04.2003**
(21) Application number: 02425524.2
(22) Date of filing: 09.08.2002
(51) Int. Cl.: A61K 9/00, A61K 31/58

(54) **Pharmaceutical formulation of a budesonide-containing water-propylene glycol solution and method of preparing it**

(30) Priority: 16.10.2001 IT RM20010614
(71) Applicant: Italchimici SpA, 00040 Pomezia (IT)
(72) Inventor: Oricchio, Maria Antonietta, 00040 Pomezia (IT); Conti, Stefano, 00040 Pomezia (IT)
(74) Representative: Conforti Cioncoloni, Marisa

(57) **Abstract**

A pharmaceutical formulation of a budesonide-containing water-propylene glycol solution is composed of budesonide, as an active substance, in a concentration between 0.05 and 0.1%, a stabilizer of sodium chloride, citric acid and sodium citrate, in an amount between 0.005 and 0.5%, and a solvent of polypropylene glycol and water, in the required amount to achieve 100% of a finished solution. A method of preparing such a solution is also described.

## Description

The present invention relates to a pharmaceutical formulation of a budesonide-containing water-propylene glycol solution. Further, it concerns a method of preparing such a solution. Budesonide is a corticosteroid (a synthetic non-halogenated glucocorticoid) that is structurally similar to 16-alpha-hydroxyprednisolone. Budesonide is provided with a topically effective, strong antiinflammatory activity and a low systemic activity. For this reason budesonide is used as an active substance in a multiplicity of pharmaceutical formulations as a locally-applied remedy for the treatment of both nasal and bronchial disorders.

By virtue of the chemical and physical characteristics of budesonide, that can be readily crystallized also as microcrystals, has a low melting point and is insoluble in water, budesonide has been prepared until now in the form of aqueous suspensions, with or without preservatives.

As known, the suspensions are mixtures composed of two phases, i.e. a solid phase dispersed in a liquid. The solid phase comprising budesonide tends in time to deposit from suspension onto the bottom of its container. Therefore, the suspension, to be kept as such, needs chemical additives or vigorous stirring.

For this reason the budesonide suspension is not suitable to be delivered by an electric nebulizer for topical administration to the respiratory system of a patient.

An objective of the present invention is to make a preparation containing budesonide, as an active substance, so that the preparation constitutes an inalterable in time, uniform molecular dispersion throughout the preparation.

Another objective of the invention is to allow a preparation containing budesonide as an active substance to be delivered by an electric nebulizer for respiratory topical administration.

Therefore, according to a first aspect of the present invention, there is provided a pharmaceutical formulation of a budesonide-containing water-propylene glycol solution, characterized by the following composition:
- budesonide, as an active substance, in a concentration between 0.05 and 0.1%;
- a stabilizer, composed of at least one substance selected from the group consisting of sodium chloride, citric acid and sodium citrate, in an amount between 0.005 and 0.5%;
- a solvent of polypropylene glycol and water, in the required amount to achieve 100% of a finished solution.

According to a second aspect of the present invention, there is provided a method of preparing a budesonide-containing water-propylene glycol solution, comprising the following steps:
- dissolving budesonide in a suitable reactor in a required amount of propylene glycol by stirring, in order to obtain a propylene glycol solution;
- dissolving substances that are included in the group of stabilizer comprising sodium chloride, citric acid and sodium citrate in a suitable reservoir in 75% of the required water, in order to obtain an aqueous solution;
- transferring the aqueous solution to the propylene glycol solution under stirring and nitrogen stream, to obtain a sole solution;
- determining the pH of the sole solution and using, if any, citric acid at 20% to adjust it;
- diluting yet to volume under stirring and nitrogen stream, to obtain the finished solution;
- filtering the finished solution;
- finally packaging the finished solution.

An advantage of the invention is that the solution obtained is composed of a uniform dispersion of a solute in a solvent to constitute one phase that remains inalterable in time. The solution allows the active substance to be administered constantly in time by a nebulizer.

An embodiment of the method of preparing a budesonide-containing water-propylene glycol solution according to the present invention is explained below.

In a suitable reactor, such an amount of budesonide, so that a concentration between 0.05 and 0.1%, for example 0.07%, will be obtained in a final solution, is dissolved in the required amount of propylene glycol by stirring, in order to obtain a propylene glycol solution.

A stabilizer comprising at least one of the substances included in the group of sodium chloride, citric acid and sodium citrate is dissolved in a suitable reservoir in 75% of the required water, in order to obtain an aqueous solution. The amount of stabilizer is between 0.005 and 0.5%, e.g. 0.05%. The mutual rate of polypropylene glycol to water is variable from 30/70 to 70/30, in the required amount to achieve 100% of a finished solution.

Then, the aqueous solution is transferred to the propylene glycol solution under stirring and nitrogen stream, to obtain a sole solution.

The pH of this sole solution is determined. If the pH is not the desired one, citric acid at 20% is used to adjust it.

The sole, water-propylene glycol solution, is diluted to volume yet under stirring and nitrogen stream, to obtain the finished solution.

The finished solution is filtered by a filter with a mesh of 0.22 µm and then is packaged in high-density polyethylene containers having different capacities, from 15 to 40 ml.

## Claims

1. A pharmaceutical formulation of a budesonide-containing water-propylene glycol solution, **characterized by** the following composition:
- budesonide, as an active substance, in a concentration between 0.05 and 0.1%;
- a stabilizer, composed of at least one substance selected from the group consisting of sodium chloride, citric acid and sodium citrate, in an amount between 0.005 and 0.5%;
- a solvent of polypropylene glycol and water, in the required amount to achieve 100% of a finished solution.

2. The pharmaceutical formulation according to claim 1, **characterized in that** budesonide is in a concentration of 0.7% with respect to the finished solution.

3. The pharmaceutical formulation according to claim 1, **characterized in that** the stabilizer is in a concentration of 0.05% with respect to the finished solution.

4. The pharmaceutical formulation according to claim 1, **characterized in that** the mutual rate of polypropylene glycol to water is variable from 30/70 to 70/30.

5. A method of preparing a budesonide-containing water-propylene glycol solution according to claim 1, **characterized in that** the method comprises the following steps:
- dissolving budesonide in a suitable reactor in a required amount of propylene glycol by stirring, in order to obtain a propylene glycol solution;
- dissolving substances that are included in the group of stabilizer comprising sodium chloride, citric acid and sodium citrate in a suitable reservoir in 75% of the required water, in order to obtain an aqueous solution;
- transferring the aqueous solution to the propylene glycol solution under stirring and nitrogen stream, to obtain a sole solution;
- determining the pH of the sole solution and using, if any, citric acid at 20% to adjust it;
- diluting to volume yet under stirring and nitrogen stream, to obtain the finished solution;
- filtering the finished solution;
- finally packaging the finished solution.
